# EUROPÄISCHE PATENTSCHRIFT

(11) **EP 0 785 929 B1**
(45) Veröffentlichungstag und Bekanntmachung des Hinweises auf die Patenterteilung: **12.05.1999**
(21) Anmeldenummer: 95935434.1
(22) Anmeldetag: 05.10.1995
(51) Int. Cl.: C07D 301/16, C07D 303/42

(54) **VERFAHREN ZUR HERSTELLUNG VON EPOXIDGRUPPEN ENTHALTENDEN ORGANISCHEN VERBINDUNGEN**
PROCESS FOR PREPARING EPOXIDE GROUP-CONTAINING ORGANIC COMPOUNDS
PROCEDE DE PREPARATION DE COMPOSES ORGANIQUES CONTENANT DES GROUPES EPOXYDES

(30) Priorität: 14.10.1994 DE 4436760
(43) Veröffentlichungstag der Anmeldung: 30.07.1997
(73) Patentinhaber: Henkel Kommanditgesellschaft auf Aktien, 40191 Düsseldorf (DE)
(72) Erfinder: BOHLANDER, Ralf, D-40599 Düsseldorf (DE); RIDINGER, Richard, D-40589 Düsseldorf (DE); KIENAST, Hermann, D-40764 Langenfeld (DE)
(86) Internationale Anmeldenummer: EP9503930
(87) Internationale Veröffentlichungsnummer: WO9611919

(56) Entgegenhaltungen:
- EP-A- 0 032 989
- EP-A- 0 032 990
- EP-A- 0 286 937
- DE-B- 1 185 189
- US-A- 2 774 774
- US-A- 5 268 493

## Beschreibung

### Gegenstand der Erfindung

Die vorliegende Erfindung betrifft ein Verfahren zur Herstellung von Epoxidgruppen enthaltenden organischen Verbindungen, worin man einfach oder mehrfach ungesättigte organische Verbindungen mit einer wäßrigen Wasserstoffperoxidlösung in Gegenwart von Carbonsäuren oxidiert.

### Stand der Technik

Die Epoxidierung von ungesättigten organischen Verbindungen wird in der Literatur vielfach beschrieben. Aus der **US-PS-2,774,774** ist ein Verfahren zur Epoxidierung von olefinisch ungesättigten höheren Fettsäuren bekannt, bei dem diese Säuren mit einem inerten organischen Lösungsmittel und Ameisensäure versetzt werden und dann das Gemisch auf eine Temperatur zwischen etwa 50 und 80 °C erwärmt und mit wäßrigem Wasserstoffperoxid versetzt wird. Das Reaktionsgemisch wird dabei nicht länger als etwa 5 Stunden auf eine Temperatur zwischen 50 und 80 °C gehalten. Die Ameisensäure wird in einer Menge zwischen 0,25 und 1,0 Mol und das Wasserstoffperoxid in einer Menge von etwa 1 Mol, jeweils pro Mol zu epoxidierender Doppelbindung, eingesetzt. In der **DE-OS-1 568 016** wird ein Verfahren beschrieben, bei dem α-Epoxide durch Umsetzung in einem mit Wasser nicht mischbaren Lösungsmittel mit einer in situ in einer wäßrigen Phase gebildeten alyphatischen Persäure epoxidiert werden. Bei diesem Verfahren muß das Reaktionsgemisch in einer besonderen Weise gerührt werden, so daß zwischen den beiden Phasen eine einzige Zwischenfläche aufrecht erhalten wird. Eine Dispergierung der beiden Phasen unter Tröpfchenbildung ist unbedingt zu vermeiden. Im allgemeinen wird ein Katalysator für die Bildung der alyphatischen Persäure, z.B. Schwefelsäure, mit verwendet.

Infolge der Verwendung eines Lösungsmittels und der erforderlichen besonderen Vorsichtsmaßnahmen beim Rühren ist das beschriebene Verfahren recht aufwendig und erfordert zur Erzielung annehmbarer Ausbeuten sehr lange Reaktionszeiten von bis zu 28 Stunden.

Ein übliches Verfahren besteht ferner darin, die ungesättigte organische Verbindung mit wäßriger Wasserstoffperoxidlösung in Gegenwart von Carbonsäuren umzusetzen, wobei die Carbonsäuren als Sauerstoffüberträger wirken (sogenanntes In-situ-Persäureverfahren), wie es beispielsweise in der deutschen Patentschrift **DE-PS-1 075 614** beschrieben wird. In der deutschen Patentschrift **DE-PS-1 185 189** wird das Persäureverfahren dahingehend abgewandelt, daß ein Aktivator, nämlich 1-Oxyethan-1,1-Diphosphonsäure, zugesetzt wird. Aus der Europäischen Patentschrift **EP-B-0 032 989** ist ein Verfahren zur Herstellung von α-Epoxiden mit 11 bis 24 Kohlenstoffatomen bekannt, worin das Wasserstoffperoxid und die als Sauerstoffträger eingesetzte Ameisensäure stufenweise zum Reaktionsgemisch zugegeben werden, um die Ausbeute des Epoxids zu erhöhen.

Aus der Literatur sind auch Verfahren zur Epoxidierung von Olefinen mit Persäuren oder Wasserstoffperoxid bekannt, worin Katalysatoren eingesetzt werden. Als Katalysatoren werden Nickel oder Nickel enthaltende Legierungen, lösliche Katalysatorsysteme auf der Basis von Elementen der Gruppen IV, V und VI B des Periodensystems (Ti, V, Mo, W) in Verbindung mit Elementen aus der Gruppe von Pb, Sn, As, Sb, Bi, Hg usw. beschrieben. Es können auch Katalysatoren auf Basis von Wolfram, Molybdän, Arsen oder Bor eingesetzt werden. Verfahren, worin Katalysatoren eingesetzt werden, sind beispielsweise in den deutschen Offenlegungsschriften **DE-OS-28 06 144** und **DE-OS-28 03 791** sowie in der deutschen Patentschrift **DE-PS-30 27 349** beschrieben.

Die aus dem Stand der Technik beschriebenen Verfahrensvarianten zur Herstellung von Epoxiden weisen verschiedene Nachteile auf. Verfahren, die Katalysatoren und/oder Lösungsmittel verwenden, enthalten diese in der Regel auch in den Endprodukten, so daß diese Produkte für Anwendungen in der Lebensmittelindustrie nicht geeignet sind. Auf der anderen Seite sind die Ausbeuten, die bei der Durchführung der Persäure-Verfahren erreicht werden, nicht immer zufriedenstellend. Die Durchführung eines Stufenverfahrens ist aufwendig und somit kostenintensiv.

Der vorliegenden Erfindung lag somit die Aufgabe zugrunde, ein Verfahren zur Herstellung von Epoxidgruppen enthaltender organischer Verbindungen zur Verfügung zu stellen, welches in einfacher Weise durchgeführt werden kann und hohe Ausbeuten liefert.

Gegenstand der Erfindung ist demgemäß ein Verfahren zur Herstellung von Epoxidgruppen enthaltenden organischen Verbindungen, worin man einfach oder mehrfach ungesättigte organische Verbindungen mit einer wäßrigen Wasserstoffperoxidlösung in Gegenwart von Carbonsäuren oxidiert, welches dadurch gekennzeichnet ist, daß man dem Reaktionsgemisch 0,01 bis 0,5 Gew.-%, bezogen auf die Menge der ungesättigten Verbindungen, eines Antioxidationsmittels zusetzt und während der Reaktion Inertgas durch das Reaktionsgefäß leitet.

Überraschenderweise wurde gefunden, daß sich gegenüber den oben beschriebenen, bekannten Verfahren durch eine Synergie von zugegebenen Antioxidationsmitteln und in das Reaktionsgemisch eingeleitetem Inertgas erhöhte Raum/Zeit-Ausbeuten bzw. Umsätze an Epoxid erhalten werden können. Das erfindungsgemäße Verfahren kann ohne organische Lösungsmittel und bei geeigneten Temperaturen auch ohne Verdunstungskühlung durchgeführt werden. Die erhaltenen Reaktionsprodukte sind frei von Lösungsmittel- und Übergangsmetallspuren und können somit in der Lebensmitteliundustrie eingesetzt werden. Ferner wurde festgestellt, daß sich deutlich niedrigere Produktviscositäten ergeben können, was die verfahrenstechnische Handhabung, insbesondere im industriellen Maßstab, bedeutend erleichtert.

Als geeignete ungesättigte Ausgangsverbindungen, die zur Herstellung der Epoxidverbindungen eingesetzt werden können, kommen zum Beispiel in Betracht:
Olefinkohlenwasserstoffe, wie Penten, Octen, Dodecen, Octadecen, Squalen, Derivate ungesättigter höhermolekularer Alkohole, wie die Ester der durch Reduktion natürlich vorkommender pflanzlicher oder tierischer ungesättigter Fettsäuren unter Erhaltung der Doppelbindung gewonnenen Alkohole, oder Ester ungesättigter Alkohole, welche durch Spaltung natürlicher Wachsester erhalten werden, wie Ester der ungesättigten Fettalkohole mit 14 bis 18 Kohlenstoffatomen im Molekül, insbesondere des Oleylalkohols und anderer einfach oder mehrfach ungesättigter Alkohole mit beliebigen niedrig oder höhermolekularen Carbonsäuren. Weiter kommen in Betracht entsprechende Ether der ungesättigten Alkohole mit vorzugsweise niedermolekularen Alkoholen. Ferner kann man als Ausgangsstoffe Derivate ungesättigter höhermolekularer Fettsäuren, wie z.B. deren Ester oder Amide, verwenden. Besonders geeignet sind die natürlich vorkommenden Glyceride, deren Fettsäureanteil ein- oder mehrfach ungesättigt sein kann, wie halbtrocknende Öle, vor allem Sojaöl, Baumwollsaatöl, Erdnußöl und Leinöl. Ferner sind Olivenöl und Klauenöl geeignet. Als Veresterungsalkohole für die ungesättigten Fettsäuren sind zu nennen: Ethyl-, Isopropyl-, n-Butyl-, sekundär-Butyl-, tertiär-Butyl-, tertiär-Amyl-, n-Octyl-, 2-Ethylhexyl-, Dodecyl-, Octadecyl-, Cyclohexyl-, Methylcyclohexyl-, Naphthenyl- und Benzylalkohol, mehrwertige Alkohole, wie Ethylenglykol, 1,2-Propylenglykol, 2-Ethylhexandiol-1,3, Butandiol-1,3, Butandiol-1,4, Dodekandiol-1,12, Glycerin, Pentaerythrit, Polyalkylenglykole, wie Diethylenglykol. Auch Ester aus ungesättigten Carbonsäuren und Alkoholgemischen lassen sich als Ausgangsverbindung verwenden sowie gemischte Ester aus mehrwertigen Alkoholen und verschiedenen ungesättigten Carbonsäuren, wie z.B. der Mischester aus Ethylenglykol mit Ölsäure und Leinölfettsäure. Schließlich sind auch Ester brauchbar, bei denen sowohl der Säure- als auch der Alkoholanteil ein- oder mehrfach ungesättigten Kohlenwasserstoffrest besitzt.

Neben den Estern können als Ausgangsstoffe auch Amide von ungesättigten Fettsäuren Verwendung finden, die Amidierungsprodukte ungesättigter Fettsäuren oder Fettsäuregemische mit Ammoniak, Dimethylamin, Dodecylamin, Oleylamin, Ethylendiamin, Cyclohexylamin, Benzylamin.

Das Verfahren ist naturgemäß auch auf epoxydierbare Verbindungen nicht aliphatischen Charakters anwendbar, insbesondere auf ungesättigte cycloaliphatische oder heterocyclische Verbindungen. Als Ausgangsstoffe sind hier beispielsweise zu nennen: Tetrahydrobenzolsäure- und Phthalsäureabkömmlinge oder Kondensationsprodukte nach Diels-Alder aus Dienkomponenten mit wenigstens zwei konjugierten Doppelbindungen und dienophilen Komponenten mit mindestens einer Mehrfachbindung.

Das Wasserstoffperoxid wird vorzugsweise in wäßriger Lösung eingesetzt. Es kann in verdünnter oder konzentrierter Lösung verwendet werden. Insgesamt sollte das Wasserstoffperoxid in einer Menge von mindestens 1 Mol pro Mol zu epoxidierender Doppelbindung eingesetzt werden. Bevorzugt werden pro Mol zu epoxidierender Doppelbindung 1 bis 2 Mol, besonders bevorzugt 1,1 bis 1,5 Mol Wasserstoffperoxid eingesetzt. Das Wasserstoffperoxid kann auf einmal zur ungesättigten Verbindung zugegeben werden, in einer bevorzugten Ausführungsform wird es jedoch allmählich, über den gesamten Reaktionszeitraum, d.h. im Sinne einer kontinuierlichen Verfahrensführung, zugesetzt.

Das eigentliche Oxidationsmittel, die Persäure, wird in Situ durch Reaktion von Wasserstoffperoxid mit der Carbonsäure gebildet. Als Carbonsäuren kommen insbesondere solche mit 1 bis 4 Kohlenstoffatomen in Betracht, wobei Ameisensäure und Essigsäure bevorzugt eingesetzt werden. Die Carbonsäure kann in einer Menge von 0,05 bis 0,8 Mol, bevorzugt 0,1 bis 0,5 Mol, pro Mol zu epoxidierender Doppelbindung eingesetzt werden.

Erfindungsgemäß wird dem Reaktionsgemisch 0,05 bis 0,5 Gew.-%, bezogen auf die ungesättigten Verbindungen, eines Antioxidationsmittels zugesetzt. Als Antioxidationsmittel kommnen insbesondere Hydrochinon, Dimethylformamid, tert.-Butylhydroxytoluol (BHT), tert.-Butylhydroxyanisol (BHA), Tocopherol und Tocopherolacetat in Betracht. Besonders bevorzugt wird Tocopherol, das in natürlichen Ölen bereits enthalten ist, zugesetzt.

Während der Reaktion wird erfindungsgemäß ein Inertgas durch das Reaktionsgemisch geleitet. Das Inertgas wird bevorzugt mit einer Geschwindigkeit von 0,02 - 0,4 m³/h pro m³ Reaktionsgemisch durch das Reaktionsgemisch geleitet. Es durchströmt das Reaktionsgemisch und bewirkt somit eine Durchmischung der wäßrigen und organischen Phase. Das Inertgas wird in einer solchen Menge eingeleitet, daß O₂ und Ozon entfernt werden. Als Inertgase kommen Argon und vorzugsweise Stickstoff in Betracht.

Zur Durchführung des erfindungsgemäßen Verfahrens ist es bevorzugt, daß die ungesättigte organische Verbindung und die Carbonsäure in einem Reaktionsgefäß unter Rühren vorgelegt werden. Zum vorgelegten Gemisch aus ungesättigter Verbindung und Carbonsäure wird allmählich die Wasserstoffperoxidlösung zugegeben. Da die Oxidation der Doppelbindungen eine exotherme Reaktion darstellt, kann es angebracht sein, das Reaktionsgemisch durch eine extern angelegte Kühlung zu kühlen. Die Reaktionstemperatur liegt bevorzugt zwischen 0 und 100 °C, besonders bevorzugt zwischen 30 und 70 °C.

Es ist möglich, dem Reaktionsgemisch einen Aktivator/Stabilisator für die Persäurebildung zuzusetzen. Als derartige Aktivatoren kommen insbesondere Oxysäuren in Betracht, z. B. Phosphorsäure, Acetophosphonsäure, Schwefelsäure, Hydroxyethandiphosphonsäure (HEDP) oder Azacycloheptandiphosphorsäure (AHP). Die Oxysäuren können im Reaktionsgemisch belassen werden.

Das Ende der Reaktion kann durch Bestimmung des Epoxidgehaltes des Reaktionsgemisches festgestellt werden. Die Reaktionsdauer beträgt üblicherweise von 1 bis 10 Stunden. Nach Beendigung der Reaktion läßt man das Reaktionsgemisch noch über einen Zeitraum von 0,5 bis 1 Stunde nachreagieren. Die Isolierung des Reaktionsproduktes erfolgt nach Phasentrennung durch einfache Abtrennung der organischen Phase. Wie dem Fachmann geläufig ist, ist es dabei von Vorteil, die organische Phase mit Wasser und/oder wäßriger Natriumbicarbonatlösung neutral zu waschen.

Die organische Phase kann dann in an sich bekannter Weise im Vakuum unter leichtem Erwärmen getrocknet werden. Die nach dem erfindungsgemäßen Verfahren hergestellten Epoxide können direkt ohne weitere Reinigung weiter verwendet werden. Sollte eine weitere Reinigung in speziellen Fällen erwünscht sein, können sie aber auch in an sich bekannter Weise, z.B. durch Destillation unter vermindertem Druck, weiter gereinigt werden.

Die nach der Abtrennung der organischen Phase wäßrige Phase kann in an sich bekannter Weise zur Rückgewinnung des enthaltenen Wasserstoffperoxids aufgearbeitet oder aber verworfen werden.

Das erfindungsgemäße Verfahren soll in den nachfolgenden Beispielen näher erläutert werden, ohne darauf beschränkt zu werden.

### Beispiele

### Verfahrensführung

Die ungesättigte organische Verbindung und die Carbonsäure wurden vorgelegt und gerührt und auf Reaktionstemperatur erwärmt. Eine Mischung aus Wasserstoffperoxid und Säure wurde unter Rühren zugetropft.

Der Gehalt der ungesättigten Anteile in den organischen Ausgangsverbindungen und Endprodukten wurde per Jodzahltitration bestimmt (DGF C-V 11a). Der Epoxidgehalt wurde mittels EpO-Titration nach Jay ermittelt. Bei leicht flüchtigen Verbindungen wurde das Verhältnis Olefin/-Epoxid zusätzlich gaschromatographisch bestimmt. Aus dem Verhältnis von Jodzahl (Olefin) zu Epoxid-Wert (Epoxid) ergibt sich der Umsetzungsgrad in Prozent.

Nach beendeter Reaktion wurde eine Phasentrennung durchgeführt. Die wäßrige Phase wurde verworfen. Die organische Phase wurde mit gesättigter wäßriger Natriumbicarbonatlösung und Wasser neutral gewaschen, im Vakuum bei 100 °C getrocknet, wobei eventuell vorhandenes Lösungsmittel abdestilliert wurde. Anschließend wurde über 1 % Filterhilfsmittel filtriert.

### 1. Ausgangssubstanz: raffinertes Leinöl

A.
   500 g Leinöl (Iodzahl 190)
   35 g 85%ige Ameisensäure (0,17 rel. mol)
   230 g H₂O₂ (70%ig) (1,27 rel. mol)
   5 g HEDP (50%ig)

   Das H₂O₂ wurde bei 40 bis 50 °C über 1 Std. zum Reaktionsgemisch aus den oben angegebenen Komponenten getropft.
   Man ließ 1 Std. nachrühren. Die Mischung wurde zunehmend dickflüssiger. Die Aufarbeitung erfolgte mit Sulfat-haltigem Wasser wie oben beschrieben.
B. Die Reaktion wurde wie in A durchgeführt, mit der Ausnahme, daß das Leinöl in 500 g Benzol gelöst wurde. Das Reaktionsgemisch blieb dünnflüssig. Benzol wurde nach dem Trocknen bei 50°C unter leichtem Vakuum destillativ entfernt.
C. wie A, Zugabe von 0,1 g Coviox^{(R)} (natürliches Vitamin E, im Handel erhältlich von Henkel/Grünau)
D. wie A, Durchleiten von N₂-Gas von Anfang der Reaktion an.
E. (erfindungsgemäß) wie 1, Zugabe von 0,1 g Coviox^{(R)} und Durchleiten von N₂-Gas von Anfang der Reaktion an.

Die Versuchsergebnisse sind in Tabelle 1 wiedergegeben.

**Tabelle 1**

| | **Iodzahl n.Kaufmann** | **% Epoxid n. Jay** | **Viskosität n. Höppler (mPas)** |
|---|---|---|---|
| A | 2 | 6,2 | 5500 |
| B | 3 | 9,2 | 1200 |
| C | 3 | 6,7 | 1800 |
| D | 2 | 8,2 | 1500 |
| E (gemäß der Erfindung) | 2 | 9,3 | 1100 |

### 2. Ausgangssubstanz: raffinertes Sojaöl

A.
   500 g Sojaöl (Iodzahl 130)
   500 g Lösungsmittel (Solvesso^{(R)}, Gemisch aus aromatischen Kohlenwasserstoffen, im Handel erhältlich von Shell AG)
   25 g 85%ige Ameisensäure (0,18 rel. mol)
   210 g H₂O₂ (50%ig) (1,20 rel. mol)
   1 g Phosporsäure (85 %ig)

   Das H₂O₂ wurde bei 50 bis 60 °C über 1 Std. zum Reaktionsgemisch aus den oben angegebenen Komponenten getropft. Die organische Phase färbte sich dabei rötlich. Man ließ 5 Std. bei 60 °C nachrühren, es erfolgte ein Farbumschlag nach gelb. Die Aufarbeitung erfolgte mit Sulfat-haltigem Wasser wie oben beschrieben.
B. Die Reaktion wurde wie in A durchgeführt, dabei wurde die Nachrührzeit auf 8 Std. verlängert.
C. wie A, ohne Zusatz von Lösungsmittel.
D. wie B, ohne Zusatz von Lösungsmittel.
E. (erfindungsgemäß) wie 4, Zugabe von 0,5 g Coviox^{(R)} und Durchleiten von Argon von Anfang der Reaktion an.

Die Versuchsergebnisse sind in Tabelle 2 wiedergegeben.

**Tabelle 2**

| | **Iodzahl n. Kaufmann** | **% Epoxid n. Jay** | **Viskosität n. Höppler (mPas)** |
|---|---|---|---|
| A | 4,5 | 6,5 | 480 |
| B | 3,7 | 6,7 | 460 |
| C | 5,3 | 6,3 | 530 |
| D | 4,8 | 6,5 | 490 |
| E (gemäß der Erfindung) | 3,5 | 6,8 | 450 |

### 3. Ausgangssubstanz: Dioleyltetrahydrophthalat

A.
   700 g Dioleyltetrahydrophthalat (Dotp) (Kennzahlen: Säurezahl 0,3; Verseifungszahl 160; OH-Zahl 10; Iodzahl 120)
   60 g H₂O₂ (50%ig)
   5 g Phosphorsäure (85 %ig)
   580 g H₂O₂ (50%ig)

   Das H₂O₂ wurde bei 50 °C über 1 Std. zum Reaktionsgemisch aus den oben angegebenen Komponenten getropft. Man ließ 10 Std. bei 60 °C nachrühren. Die Aufarbeitung erfolgte wie oben beschrieben.
B. wie A, Zusatz von 5 g α-Naphthol
C. wie B, zusätzlich Durchleiten von N₂-Gas

Die Versuchsergebnisse sind in Tabelle 3 wiedergegeben.

**Tabelle 3**

| | **Iodzahl n. Kaufmann** | **% Epoxid n. Jay** | **Viskosität n. Höppler (mPas)** |
|---|---|---|---|
| A | 10 | 5,3 | 460 |
| B | 10 | 5,4 | 450 |
| C (gemäß der Erfindung) | 9 | 5,5 | 430 |

### 4. Ausgangssubstanz: α-C₁₂-Olefin

Der Versuch wurde in 3 Verfahrensstufen durchgeführt. Nach jeder Verfahrensstufe wurde die wäßrige Phase abgezogen.
A.
843 g C₁₂-Olefin (5 mol) (Iodzahl 152)
82 g 85%ige Ameisensäure (0,3 mol)
366 g H₂O₂ (70%ig) (1,5 mol)
3 g Acetophosphonsäure (60 %ig)

Die Gesamtmenge an Ameisensäure, H₂O₂ und Acetophosphonsäure wurden aufgeteilt:
a. 40 g Ameisensäure zum Olefin dosiert und 185 g H₂O₂ und 2 g Säure wurde über 1 Std. bei 60°C zugetropft, man ließ 10 Std. nachrühren. Die saure wäßrige Phase wurde abgezogen und verworfen.
b. 21 g Ameisensäure wurden zum Olefin dosiert und 92 g H₂O₂ und 0,5 g Säure über 30 min. bei 60°C zugetropft. Man ließ 5 Std. nachrühren. Die saure wäßrige Phase wurd abgezogen und verworfen.
c. wie b.
Versuchsergebnis:

| | **% Olefin** | **% Epoxid (gaschromatographisch)** |
|---|---|---|
| **a** | 60 | 38 |
| **b** | 33 | 64 |
| **c** | 10 | 83 |

B. (erfindungsgemäß) wie A, Zusatz von 1 g Dimethylformamid und Durchleiten eines leichten Stickstoffstroms
Versuchsergebnis:

| | **% Olefin** | **% Epoxid (gaschromatographisch)** |
|---|---|---|
| **a** | 59 | 39 |
| **b** | 30 | 67 |
| **c** | 5 | 90 |

Aus den voranstehenden Ergebnissen wird deutlich, daß mit der erfindungsgemäßen Verfahrensführung eine höhere Epoxidausbeute erhalten wird. Die Versuche 1 bis 3 zeigen gleichzeitig, daß das Reaktiosgemisch eine niedrigere Viskosität aufweist, was für die Weiterverarbeitung vorteilhaft ist.

## Patentansprüche

1. Verfahren zur Herstellung von Epoxidgruppen enthaltenden organischen Verbindungen, worin man einfach oder mehrfach ungesättigte organische Verbindungen mit wäßriger Wasserstoffperoxidlösung in Gegenwart von Carbonsäuren oxidiert, dadurch gekennzeichnet, daß man dem Reaktionsgemisch 0,01 bis 0,5 Gew.-%, bezogen auf die Menge der ungesättigten Verbindungen, eines Antioxidationsmittels zusetzt, und während der Reaktion Inertgas durch das Reaktionsgefäß leitet.

2. Verfahren nach Anspruch 1, dadurch gekennzeichnet, daß man als ungesättigte organische Verbindungen α-Olefine und ungesättigte Öle nativen Ursprungs einsetzt.

3. Verfahren nach einem der Ansprüche 1 oder 2, dadurch gekennzeichnet, daß man als Antioxidationsmittel Hydrochinon, Dimethylformamid, tert.-Butylhydroxytoluol, tert-Butylhydroxyanisol, Tocopherol und Tocopherolacetat einsetzt.

4. Verfahren nach einem der Ansprüche 1 bis 3, dadurch gekennzeichnet, daß man das Inertgas mit einer Geschwindigkeit von 0,02 - 0,4 m³/h pro m³ Reaktionsgemisch durch das Reaktionsgemisch leitet.

5. Verfahren nach einem der Ansprüche 1 bis 4, dadurch gekennzeichnet, daß man die Reaktion ohne Lösungsmittel durchführt.

6. Verfahren nach einem der Ansprüche 1 bis 6, dadurch gekennzeichnet, daß man die Reaktion bei einer Temperatur von 0 bis 100 °C durchführt.

7. Verfahren nach einem der Ansprüche 1 bis 6, dadurch gekennzeichnet, daß man das Wasserstoffperoxid in einer Menge von 1 bis 2 Mol pro Mol zu epoxidierender Doppelbindung zugibt.

8. Verfahren nach einem der Ansprüche 1 bis 7, dadurch gekennzeichnet, daß man als Carbonsäure Ameisensäure, Essigsäure, Chloressigsäure, Propionsäure, Benzoesäure oder deren Halogenierungsprodukte einsetzt.

9. Verfahren nach einem der Ansprüche 1 bis 8, dadurch gekennzeichnet, daß man die Reaktion in Gegenwart eines Aktivators durchführt.

10. Verfahren nach Anspruch 9, dadurch gekennzeichnet, daß man als Aktivator eine Oxysäure einsetzt.

## Claims

1. A process for the production of organic compounds containing epoxide groups, in which mono- or polyunsaturated organic compounds are oxidized with aqueous hydrogen peroxide solution in the presence of carboxylic acids, characterized in that 0.01 to 0.5% by weight, based on the quantity of unsaturated compounds, of an antioxidant is added to the reaction mixture and inert gas is passed through the reaction vessel during the reaction.

2. A process as claimed in claim 1, characterized in that α-olefins and unsaturated oils of natural origin are used as the unsaturated organic compounds.

3. A process as claimed in claim 1 or 2, characterized in that hydroquinone, dimethyl formamide, tert.-butyl hydroxytoluene, tert.butyl hydroxyanisole, tocopherol and tocopherol acetate are used as the antioxidant.

4. A process as claimed in any of claims 1 to 3, characterized in that the inert gas is passed through the reaction mixture at a rate of 0.02 to 0.4 m³/h per m³ reaction mixture.

5. A process as claimed in any of claims 1 to 4, characterized in that the reaction is carried out without solvents.

6. A process as claimed in any of claims 1 to 5, characterized in that the reaction is carried out at a temperature of 0 to 100°C.

7. A process as claimed in any of claims 1 to 6, characterized in that the hydrogen peroxide is added in a quantity of 1 to 2 moles per mole of double bond to be epoxidized.

8. A process as claimed in any of claims 1 to 7, characterized in that formic acid, acetic acid, chloroacetic acid, propionic acid, benzoic acid or halogenation products thereof is/are used as the carboxylic acid.

9. A process as claimed in any of claims 1 to 8, characterized in that the reaction is carried out in the presence of an activator.

10. A process as claimed in claim 9, characterized in that an oxyacid is used as the activator.

## Revendications

1. Procédé de production de composés organiques contenant des groupes époxyde, dans lequel on oxyde des composés organiques une fois ou plusieurs fois non saturés avec une solution aqueuse de peroxyde d'hydrogène en présence d'acides carboxyliques,
caractérisé en ce qu'
on ajoute au mélange réactionnel de 0,01 à 0,5 % en poids, rapporté à la quantité de composés non saturés, un agent antioxydant et en ce que l'on fait passer pendant la réaction un gaz inerte à travers le récipient de réaction.

2. Procédé selon la revendication 1,
caractérisé en ce qu'
on met en oeuvre comme composés organiques non saturés, des α-oléfines et des huiles non saturées d'origine native.

3. Procédé selon l'une des revendications 1 ou 2,
caractérisé en ce qu'
on met en oeuvre comme agent antioxydant de l'hydroquinone, du diméthylformamide, du terbutylhydroxytoluène, du terbutylhydroxyanisole, du tocophérol et de l'acétate de tocophérol.

4. Procédé selon l'une des revendications 1 à 3,
caractérisé en ce qu'
on fait passer le gaz inerte avec une vitesse de 0,02 à 0,4 m³/h par m³ de mélange réactionnel au travers du mélange réactionnel.

5. Procédé selon l'une des revendications 1 à 4,
caractérisé en ce qu'
on effectue la réaction sans solvant.

6. Procédé selon l'une des revendications 1 à 5,
caractérisé en ce qu'
on effectue la réaction à une température allant de 0 à 100°C.

7. Procédé selon l'une des revendications 1 à 6,
caractérisé en ce qu'
on ajoute le peroxyde d'hydrogène en une quantité de 1 à 2 mol par mol de double liaison à époxyder.

8. Procédé selon l'une des revendications 1 à 7,
caractérisé en ce qu'
on met en oeuvre comme acide carboxylique de l'acide formique, de l'acide acétique, de l'acide chloracétique, de l'acide propionique, de l'acide benzoïque ou leurs produits d'halogénation.

9. Procédé selon l'une des revendications 1 à 8,
caractérisé en ce qu'
on effectue la réaction en présence d'un activateur.

10. Procédé selon la revendication 9,
caractérisé en ce qu'
on met en oeuvre comme activateur, un oxyacide.
